(19) Europäisches Patentamt
European Patent Office
Office européen des brevets

(11) **EP 2 338 870 A1**

(12) **EUROPEAN PATENT APPLICATION**

(43) Date of publication:
**29.06.2011 Bulletin 2011/26**

(51) Int Cl.:
**C07C 69/80** (2006.01)     **C07C 67/08** (2006.01)
**C07C 67/48** (2006.01)     **H01B 3/44** (2006.01)

(21) Application number: **10189204.0**

(22) Date of filing: **20.08.2004**

(84) Designated Contracting States:
**AT BE BG CH CY CZ DE DK EE ES FI FR GB GR HU IE IT LI LU MC NL PL PT RO SE SI SK TR**

(30) Priority: **29.08.2003 GB 0320206**

(62) Document number(s) of the earlier application(s) in accordance with Art. 76 EPC:
**04764340.8 / 1 663 940**

(71) Applicant: **ExxonMobil Chemical Patents Inc.**
**Baytown, TX 77520-2101 (US)**

(72) Inventors:
• **De Munck, Nicolaas**
**2991 JL Barendrecht (NL)**

• **Gosse, Claudius**
**3080 Tervuren (BE)**
• **Caers, Raphael, Frans**
**2650 Edegem (BE)**

(74) Representative: **Wall, Leythem**
**ExxonMobil Chemical Europe Inc.**
**IP Law Shared Services**
**Hermeslaan 2**
**1831 MACHELEN (BE)**

Remarks:
This application was filed on 28-10-2010 as a divisional application to the application mentioned under INID code 62.

(54) **Improvements in or relating to phthalate plasticiser esters**

(57) The liquid volume resistivity of plasticiser esters is improved by purifying the ester with an adsorbent having a pH in the range 6 to 11. It is preferable to use a mixture of a filtration aid and an adsorbent. A plasticiser having a desirable combination of high liquid volume resistivity, low amount of light ends and low carbonyl number is obtained.

**FIGURE 1**

**Description**

The present invention relates to improvements in or relating to plasticisers and in particular to phthalate ester plasticisers useful in polyvinyl chloride compositions.

The properties and quality requirements for plasticisers depend upon the use to which the plasticiser is to be put. One important property of a plasticiser is its electrical resistivity, particularly when it is to be used in electrical applications such as for wire and cable insulation.

More specifically, the present invention relates to a process for producing a high quality plasticiser ester suitable for use with polyvinyl chloride which is to be employed in a composition useful for wire and cable insulation and for other electrical insulating materials.

Plasticised polyvinyl chloride is widely used for insulation in the electric and electronic industries and these uses require a high-quality plasticiser ester. For example, a plasticiser having high volume resistivity is required in the electrical field. The resistivity of a plasticised polyvinyl chloride composition may be measured as the Pad Volume Resistivity (PVR). Many people in the industry also measure the resistivity of the plasticiser itself, which is known as the liquid volume resistivity (LVR) of a plasticiser. For several electrical applications, such as the electrical insulation of under-the-hood or under-the-dashboard electrical wire and cables in vehicles, plasticisers are preferred to have a high LVR, and a low amount of lights, especially those compounds that contribute to odour and automotive interior fog problems. The electrical equipment in vehicles is becoming more and more complex and sophisticated. Modem vehicles are becoming equipped with more and more sensors and electrically driven devices. The amount of wiring and cabling necessary for connecting these sensors and controlling and powering these devices continues to increase. Many of these connections are placed out of sight under the vehicle upholstery and relatively close to the outer body, where there is little ventilation and temperatures are high due to engine heat or when the vehicle is exposed to sunshine.

Plasticisers should therefore have an acceptable odour, and should not cause fogging or the creation of a light scattering film on the innerside of car windshields. They should also be resistant to ultra violet light. The plasticiser should contain a minimal amount of volatiles or light ends to have a low odour level both during its processing and in its final application.

Plasticiser esters are typically made by esterification of $C_6$ to $C_{13}$ alcohols with acid or anhydride. The alcohols themselves are in many instances made by hydroformylation of olefins. The process may involve olefin oligomerisation followed by hydroformylation and hydrogenation, or olefin hydroformylation followed by aldol condensation and hydrogenation. The starting material for the production of di-2-ethyl-hexyl phthalate is generally chemical grade propylene. However, for other alcohols the starting materials are commonly mixtures of olefins. The hydroformylation, hydrogenation and condensation reactions are all catalysed. Accordingly, in many of the steps of the processes complex reaction mixtures tend to be formed. The final alcohol therefore requires extensive purification to remove unreacted raw materials, undesirable byproducts and catalyst residues. Purification typically involves washing, further hydrogenation or hydrofinishing and fractional distillation. However, despite rigorous purification, the purified alcohols invariably contain small amounts of impurities.

The plasticiser esters are then produced by reaction of the appropriate alcohol with an acid anhydride, frequently phthalic anhydride, trimellitic anhydride, or maleic anhydride, or with an acid. Acids frequently used are adipic acid, trimellitic acid, cyclohexanoic mono- and dibasic acids, benzoic acid, citric acid and the like. The esterification is typically performed using an organometallic catalyst, particularly a titanium or tin based catalyst, but many other esterification catalysts like sulfuric acid, methyl sulfonic acid and paratoluene sulfonic acid are also known. After esterification the crude ester will contain contaminants and requires purification. These contaminants may belong to the family of acidic residues, unreacted alcohol, catalyst residues, water and the contaminants that were already present in the alcohol feed, most of these being so-called monomeric components that are eluted in the so-called "light ends" region of the plasticiser Gas Chromatogram or GC-spectrum, as discussed later. The crude esters may also contain byproducts, such as alcohol (di-alkyl) ethers, benzoate esters, mono-esters from dibasic acids, alcohol oxo acid esters, hemiacetals and vinyl ethers (these are so-called dimeric components and are often collectively called "ethers" or "intermediates" due to their elution in the plasticiser Gas Chromatogram or GC-spectrum between the monomeric light ends and the "trimeric" diesters). Many of these dimeric materials, as well as acetals which are "trimeric" compounds, may become hydrolysed during later stages in the process to form odour formers such as aldehydes and/or other light ends.

Purification involves contacting the crude ester with aqueous alkali. The addition of the alkali hydrolyses catalyst residues and neutralises any undesirable alkyl hydrogen phthalate mono-ester that may be present. The products of these hydrolysis and/or neutralisation reactions may show up either as solids or as dissolved in a separate free water phase, possibly partially in one of the mentioned forms. The free water phase may then be removed, e.g. by flashing or distillation, and the amount of solids present may be increased. Alternatively the free water phase may be removed by decanting, preferably preceded by an additional washing step for additional purification. The neutralised ester is then typically contacted with a filter aid and filtered for the removal of salts such as the alkali salts of alkyl hydrogen phthalate mono-esters, the hydroxide of the organo-metal catalyst such as titanium hydroxide, the oxide of the organo-metal catalyst such as titanium dioxide or tin oxide, and sodium (bi-) carbonate. The alkali is preferably sodium carbonate or in some instances it may be sodium hydroxide, preferably in an aqueous form, and the treatment with alkali may be followed by injection of carbon dioxide to convert any remaining sodium hydroxide into water and sodium (bi-) carbonate. Finally, any excess alcohol and water may be removed e.g. by flashing or stripping with a vapour, e.g. with steam or nitrogen, or by a combination thereof.

The series of reactions involved in the production of plasticisers therefore involves complex mixtures and catalysis giving rise to a number of byproducts and impurities. Extensive purification is therefore required to produce plasticiser esters having the properties required for their various applications. The presence of the contaminants as discussed above can have adverse effects on the properties of the plasticiser, in particular on the liquid volume resistivity and the odour. In particular the light ends can contribute to the formation of a stronger odour of the plasticiser itself or of the final product comprising it. For higher molecular weight phthalates like di-nonylphthalate (LNP), di-isononylphthalate (DINP), di-isodecylphthalate (DIDP), di-undecylphthalate (DUP), di-isoundecylphthalate (DIUP), undecyl-dodecylphthalate (UDP), and di-(iso)tridecylphthalate (DTDP), it has been found that a high level of ethers or other intermediates increases the contribution to fogging of the plasticiser. In particular the presence of the impurities has been found to lower the LVR of the plasticiser and can also reduce the stability of the plasticiser to ultra violet light.

There is a need to improve the throughput of plasticiser manufacturing facilities so that larger volumes of product may be produced in the same facility. We have, however, found that although the throughput can be increased, the increase can result in a deterioration of the electrical properties of the plasticiser as indicated by a reduction in the LVR of the plasticiser. In one aspect the present invention is concerned with overcoming this problem.

United States Patent 5,880,310 is concerned with producing plasticiser esters with high liquid volume resistivity as measured by Japanese Industry Standard JIS K-6751. United States Patent 5,880,310 obtains high volume resistivity of a plasticiser ester by blowing carbon dioxide into the crude ester that has been treated with sodium hydroxide, to convert residual alkali into a (bi-) carbonate, recovering any excess alcohol, typically by steam stripping, and then subjecting the stripped ester to a combination of fine filtration, using a filter aid, and adsorption treatment. Accordingly, the stripping is performed in the presence of solids such as sodium carbonate which are then removed during the fine filtration. Stripping with steam in the presence of alkali can cause hydrolysis of the ester, which would be expected to increase light ends. Furthermore, stripping in the presence of solids is difficult to perform in a tower-like configuration due to fouling or plugging of tower internals.

Examples of filter aids that may be used according to United States Patent 5,880,310 are a filter aid produced from diatomaceous earth which is generally available on the market [for example, Radiolite (made by Showa Kagaku Kogyo K. K.) and Celite (made by Johns Manville Sales Corp.)], and a filter aid produced from perlite [for example, Topco Perlite (made by Showa Kagaku K. K.) and Dicalite Perlite (made by Dicalite Orient K. K.)]. At least 20% of the filter aid must have a particle size of 5 microns or less.

Examples of adsorbents that may be used according to United States Patent 5,880,310 are activated alumina, activated china clay, activated carbon, magnesium oxide, aluminium oxide and silicon oxide. These may be used either singly or in combination. It is said that the amount of the adsorbent used should be between 0.1 and 1 % by weight based on the weight of the crude ester. The Examples of United States Patent 5,880,310 show, however, that the use of activated carbon as the adsorbent has no effect on the volume resistivity of the ester based on 2 ethylhexyl alcohol (see Examples 1 to 13) or on the ester based on isononyl alcohol (see Examples 14 to 22). According to the patent, the adsorbents that improve the volume resistivity when used in combination with the fine filter aid are Sekado KW (alumina silica), activated alumina and magnesium oxide.

United States Patent 6,310,235 produces esters by first reacting an acid or acid anhydride with an alcohol at from 100 to 160°C while removing water and completing the reaction by addition of a catalyst and increasing the reaction temperature to about 250°C. The reaction mixture is then neutralised with aqueous alkali metal or alkaline earth metal hydroxide. The excess alcohol is separated and the ester is dried and filtered. United States Patent 6,310,235 suggests that esters with low conductivity may be obtained by complete removal of any mono-ester, catalyst and the reagents used for neutralisation. There is however no indication of what is meant by extremely low conductivity and there is no recognition of the importance of the removal of light ends, let alone the use of purification of conditions which deter the formation of light ends and odour formers.

We have, however, found that if the filter aid and/or the adsorbent is too strongly acidic the treatment results in an increase in the light ends and a reduction of the ethers or intermediates, present in the plasticiser. The light ends and the intermediates present in the plasticiser ester may be determined by Gas Chromatography as described later. It is believed that this change in intermediates and light ends may be due to acid catalysed hydrolysis of certain impurities in the plasticiser ester. Furthermore we have found that when such filter aids and/or adsorbents that are too acidic are used, there is a tendency for the treated plasticiser to have an undesirable odour. We have also found that in a process wherein the crude ester is neutralised with sodium carbonate (as opposed to the sodium hydroxide neutralisation followed by blowing with carbon dioxide of United States Patent 5,880,310), treatment of the ester with activated carbon following neutralisation and hydrolysis results in a significant improvement in the liquid volume resistivity of the plasticiser ester.

We have found that this is particularly the case with plasticisers derived from $C_9$ to $C_{13}$ alcohols, especially $C_9$ to $C_{13}$ phthalate diesters, the $C_{10}$ to $C_{13}$ phthalate di-esters being particularly useful as plasticisers for polyvinyl chloride employed in the production of compositions useful in electrical applications such as wire and cable insulation. $I_{S0}$-$C_{13}$ phthalates are particularly useful in environments of higher temperature, such as under-the-hood wiring and cabling of vehicles. In addition we have found that there is a correlation between the extent to which the plasticiser has an undesirable odour, and the content of light ends and the carbonyl number of the plasticiser.

According to the present invention, there is provided a di-alkyl phthalate characterised by a carbonyl number below 0.2 mg KOH/g, a light ends content of less than 1000 ppm wt, and a liquid volume resistivity that is:

i) greater than $0.3 \times 10^{12}$ ohm.cm in the case where di-alkyl is di-2-ethyl hexyl;
ii) greater than $0.6 \times 10^{12}$ ohm.cm in the case where di-alkyl is di-isononyl; and
iii) greater than $1.35 \times 10^{12}$ ohm.cm in the case where di-alkyl is di-isodecyl.

In one embodiment, the invention provides di-2-ethyl-hexyl phthalate having:

i) a LVR greater than $0.3 \times 10^{12}$ ohm.cm
ii) a proportion of light ends less than 1000 ppm wt and
iii) a carbonyl number below 0.2 mg KOH/g.

Preferably the LVR of the di-2-ethyl-hexyl phthalate is greater than 0.5, more preferably greater than 0.8, especially greater than 1.0 and particularly preferred to be greater than 1.1 (x $10^{12}$ ohm.cm).
In another embodiment the invention provides di-isononyl phthalate having:

i) a LVR greater than $0.6 \times 10^{12}$ ohm.cm
ii) a proportion of light ends less than 1000 ppm wt and
iii) a carbonyl number below 0.2 mg KOH/g.

Preferably the LVR of the di-isononyl phthalate is greater than 0.7, more preferably greater than 1.0, especially greater than 1.5 and particularly preferred to be greater than 2.0 (x $10^{12}$ ohm.cm).
In yet another embodiment the invention provides a di-isodecyl phthalate having:

i) a LVR greater than $1.35 \times 10^{12}$ ohm.cm
ii) a proportion of light ends less than 1000 ppm wt and
iii) a carbonyl number below 0.2 mg KOH/g.

Preferably the LVR of the di-isodecyl phthalate is greater than 1.4, more preferably greater than 2.0, especially greater than 2.5 and particularly preferred to be greater than 3.0 (x $10^{12}$ ohm.cm)
The above phthalates are products that have not hitherto been known or attainable in the commercial field of phthalate plasticiser production.
If the Carbonyl Number is above 0.2 mg KOH/g it is believed this will have an undesirable effect on odour no matter what the light ends content. Preferably the Carbonyl Number is well below 0.2, e.g. 0.1 or below, and most preferably approaching zero.
The phthalate esters preferably have a light ends content below 600 ppm, more preferably below 500 ppm and most preferably below 200 ppm, for example from 20 to 170 ppm such as from 30 to 150 ppm. It is also independently preferred that the esters have a level of intermediates below 750 ppm, more preferably below 500 ppm and most preferably below 300 ppm, such as below 250 ppm. Preferably the esters have a combined light ends and intermediates content below 1000 ppm. All the ppm values expressed herein are by weight. The basis for the ppm wt values given is the total weight of the phthalate ester and the contaminants (light ends and/or intermediates) to which the ppm value relates.
The terms "light ends" and "intermediates" are well understood by those skilled in the art, and refer to groups of peaks in the Gas Chromatograph spectrum of a "target ester" that are quite distinct from each other and from the peak(s) corresponding to the target ester itself.
The light ends are represented by the group of peaks showing in the region where the parent alcohols of the ester elute. The target ester is represented by the peak or peaks characteristic of the target ester, including any smaller peaks that may be showing on the shoulder of the main peak or peaks. The intermediates are represented by the group of peaks in the region between those of the light ends and the target ester.
To facilitate calculation of the amounts of light ends and intermediates in a product sample, we choose to incorporate an internal standard, and have selected n-hexadecane as such internal standard. It conveniently delineates the light ends (or "monomeric") peak region from the intermediates (or "dimeric") region and permits accurate quantification of levels of light ends and intermediates. This convenience applies to most of the commercially important phthalate ester plasticiser spectra. For significantly lower or higher molecular weight phthalates than DIDP, a different internal standard may be used for convenience if needed. However, for all molecular weight plasticizer esters that are the subject of the invention, the distinctions between the light ends, the intermediates and the target ester are clear. In accordance with our studies, only those phthalate esters produced from parent alcohol mixtures that comprise alcohols with widely different molecular weights, e.g. a mix of C4 and C8 alcohols used to produce butyl octyl phthalate as target ester, run the risk of losing this distinction in the GC spectrum, and hence this ready distinction between light ends and intermediates. Such products are, however, not within the scope of the invention.

In a further embodiment the invention provides a process for the production of plasticiser esters comprising:

(i) esterifying an acid or an anhydride with an alcohol containing from 6 to 13 carbon atoms, to form a crude ester;
(ii) treating the crude ester with a base, to form a treated ester;
(iii) filtering the treated ester to separate a liquid product;
(iv) stripping the liquid product to form a stripped material;
(v) treating the stripped material with an adsorbent; and
(vi) filtering the product of step (v), optionally in the presence of a filter aid, to remove the adsorbent from the plasticiser ester.

In a further embodiment the present invention provides a method for purifying a plasticiser ester which comprises forming a mixture of the ester and an adsorbent having a pH in the range of 6 to 11, and subsequently filtering the mixture.

The pH is measured by preparing a slurry of 30 grams of the treating agent in 300 grams of deionised water and mixing for 15 minutes at room temperature. The water was deionised using reverse osmosis followed by polishing with an $H^+$ ion exchange resin and an OH- ion exchange resin, to a conductivity in the range 8 to 11 microSiemens per cm. The mixture is then filtered twice with two Macherey & Nagel MN 616 filter papers. The pH of the filtrate is then determined using a Metrohm 691 pH meter with a Metrohm 6.0202 100 electrode that has been calibrated with buffer solutions of pH 4.00; 7.00 and 10.00.

In a preferred embodiment of the method the adsorbent comprises activated carbon and the mixture also comprises a filter aid having a pH in the range 6 to 11. The pH of the filter aid is also measured using a 10 wt % slurry of the filter aid in deionised water, as described for the adsorbent.

In yet another aspect, the present invention provides the use of a mixture of activated carbon and a filter aid in the purification by filtration of a plasticiser ester, said activated carbon and filter aid each having a pH in the range 6 to 11.

Another important property of the plasticiser is that it has optical clarity and this can be affected by the presence of impurities which can cause the plasticiser to become hazy over time. We have found that one cause of haze can be the formation of salts of acids present during catalyst neutralisation with alkali. For example sodium hydrogen phthalate can be formed as a result of the neutralisation of a phthalate ester containing small amounts of phthalic acid with sodium carbonate or bicarbonate or sodium hydroxide, or from the hydrolysis of the monoester or of its salt. These compounds, being polar and capable of dissociating into ionic species, can also lead to a reduction in the LVR of the plasticiser. We have found that the purification process of the present invention can therefore also reduce the likelihood of the plasticiser forming haze during longer term storage.

The acid or anhydride employed in the process of the invention is preferably organic. Examples of the organic acid or its anhydride that may be used in the esterification reaction include aromatic monocarboxylic acids typified by benzoic acid, and folic acid; polybasic aromatic carboxylic acids or anhydrides thereof, such as phthalic acid, phthalic anhydride, isophthalic acid, terephthalic acid, trimesic acid, trimellitic acid, trimellitic anhydride, pyromellitic acid, pyromellitic anhydride, benzophenonetetracarboxylic acid and benzophenonetetracarboxylic anhydride; polybasic aliphatic carboxylic acids such as adipic acid, sebacic acid and azelaic acid and citric acid; polybasic unsaturated aliphatic carboxylic acids such as maleic acid and fumaric acid; and aliphatic monocarboxylic acids such as oleic acid and stearic acid. The various phthalic acids or anhydrides are preferred.

Examples of the alcohol that may be used in the esterification reaction include saturated monohydric aliphatic alcohols such as methanol, ethanol, propanol, isopropanol, butanol, isobutanol, sec-butanol, normal- and iso-pentanol, normal- and iso-hexanol, normal- and iso-heptanol, normal- and iso-octanol, 2-ethylhexanol, normal- and iso-nonyl alcohol, normal- and iso-decanol, 2-propyl heptanol, normal- and iso-undecanol or -dodecanol and normal- and isotridecanol; and polyhydric aliphatic alcohols such as ethylene glycol, propylene glycol and their dimers, trimers and tetramers. The alcohols may also be used in combination as required. $C_6$ to $C_{13}$ alcohols are preferably employed and the $C_9$ to $C_{11}$ alcohols, especially the $C_{10}$ and $C_{11}$ alcohols are preferred especially in the production of plasticiser esters that are used in polyvinyl chloride compositions that are used in electrical applications such as wire and cable coating.

The esterification reaction preferably employs an organic metal compound catalyst. Examples include an alkyl titanate such as tetra-isopropyl titanate, tetra-n-butyl titanate or tetra-iso-octyl titanate, or an organic tin compound such as tin oxalate, tin octanoate or tin maleate that exhibit a catalytic activity at an esterification reaction temperature.

In one embodiment of the process, the esterification reaction is conducted by adding an alcohol to an organic acid or its anhydride, and reacting the mixture, preferably at from 150°C to 220°C and preferably for from 1 to 4 hours, in the presence of an organic metal compound catalyst in an inert gas atmosphere while removing water formed. The reaction time is preferably at the lower end of the range, e.g. from 1.5 to 2 hours, and optimally even less than 1.5 hours. A base and water, preferably in the form of an aqueous base, is added to the resulting reaction solution to neutralise any unreacted acid and/or mono-ester and to hydrolyse the catalyst. It is also preferred to remove any free water after the crude ester has been treated with the base and before filtration, particularly if the treatment has been with aqueous base. Preferred bases include alkali metal salts, particularly sodium salts, like sodium carbonate, and alkali metal hydroxides, like sodium hydroxide, e.g. aqueous sodium hydroxide. Any excess alcohol is recovered typically by stripping (which advantageously removes alcohol, water and other light materials) and the resulting ester product is then purified to obtain a plasticiser.

Unlike WO 94/17028 which suggests that during their purification plasticiser esters may be subjected to a final filtration to remove solids, the purification to produce the esters of the purity of the present invention involves use of an adsorbent which removes at least some of the light ends and/or intermediates but does not catalyse the hydrolysis of byproducts to produce light ends and undesirable odour to any great extent. This treatment with adsorbent is then followed by filtration in the presence of a filter aid to remove the adsorbent. In one embodiment the adsorbent is also the filter aid. That is, a single material is employed to treat the stripped material and this also facilitates the final filtration step. In a preferred embodiment the invention involves the addition to the plasticiser ester of a combination of a filter aid and an adsorbent, in this instance the filter aid may also have an adsorbing effect. The purification is typically accomplished by the addition of the adsorbent and the filter aid (where the adsorbent is not also the filter aid) to the plasticiser ester in a stirred drum from which the plasticiser containing the adsorbent and optionally the (different) filter aid is passed to the filter, preferably a candle filter optionally equipped with a filter cloth which may be precoated with a suitable precoating material like e.g. wood flour or perlite, where the adsorbent and optionally the filter aid are retained on the filter and the purified ester is obtained as the filtrate. As mentioned, in some instances the adsorbent will also act as a filter aid.

We have found that the correct selection of the adsorbent and, where used, the filter aid, and the purification conditions are important in achieving the purification necessary to obtain the desired high LVR and to avoid the formation of undesirable light ends, odour formers and undesirable carbonyl containing compounds. We have found that, in addition, when employing the invention, the tendency of the plasticiser to form haze is reduced. One important criterion is the pH of the filter aid and the adsorbent. We have found that the pH of both materials should be between 6 and 11, preferably between 6 and 9 more preferably between 6.5 and 8.5 as measured on a 5 - 20 wt%, preferably 10 wt % slurry of the materials in dionised water. The particle size of the filter aid, when used, also contributes to performance results. Generally the smaller the particle size the better. Other criteria that have to be taken into account include the particle size distribution, internal surface area, pore size and pore volume of the adsorbent.

The time of the treatment, the temperature at which the treatment is effected and the degree of stirring have also been found, within the scope of the invention, to have an effect on the extent of purification.

Examples of materials that have been found to be useful as both filter aids and adsorbents include bleaching earths, bentonites or activated clays, comprising attapulgite or Fuller's Earth, montmorillonite, kaolinite and muskovite minerals. Some of the clay properties that may impact on performance include mineralogy, particle size distribution, surface acidity and degree of heat activation. Heat activation determines the surface area, pore volume, moisture content, and cation exchange capacity. Examples of products are Engelhard Attasorb® Attapulgite, Pure-Flo® B80 Natural and Pure-Flo® M85/20 both of Oil-Dry Corporation of America, TONSIL 3191FF (Süd-Chemie AG), VOLCANSIL RN-70 and VOLCANSIL D.E./11 (both of Bentonitas Especiales, S.A.) and frieTON frieBE. In particular, we prefer to use the product frieBE, manufactured from Friedland clay by Friedländer Ton-Industriegesellschaft mbH in Germany, which has the following typical composition and properties.

Typical chemical composition (weight):

| | | | |
|---|---|---|---|
| $SiO_2$ | 59.0 % | MgO | 2.1 % |
| $Al_2O_3$ | 19.5 % | CuO | 0.5 % |
| $Fe_2O_3$ | 6.9 % | $Na_2O$ | 0.9 % |
| $TiO_2$ | 1.0 % | $K_2O$ | 3.1 % |

Typical mineralogical composition (weight):

50 % swelling mixed - layer mineral

(continued)

| 12 % | kaolinite |
|---|---|
| 12 % | muscovite |
| 20 % | quartz |
| <6 % | feldspar, carbonates |

Typical grain size distribution:

| >75%<40 $\mu$m | |
|---|---|
| Water content: | approx 5 % wt |
| pH: | 8.3 |
| Surface area: | 185 $m^2$/g |
| Pore volume: | 0.30 ($cm^3$/g, as measured by $N_2$—adsorption) |

Another preferred and effective product is available from Süd-Chemie AG under the name TERRANA® 510, which is a natural bleaching earth manufactured by the thermal activation of calcium bentonite. Both Terrana 510 and frieTON frieBE are montmorillonites.

We prefer to use activated carbon as an adsorbent and we prefer to use the material that is commercially available as Norit SA 4G which is a steam activated carbon having the following typical properties. Alternative grades are Norit SA 4 PAH and SA 4 PAH-HF, which are similar activated carbons but differing in filtration properties.

| Molasses number (EUR) | 525 max 580 |
|---|---|
| Methylene blue adsorption | 11 g/100g |
| Iodine number (ASTM D4607) | 700 mg Iodine/g carbon |
| Total surface area (B.E.T.) | 800 $m^2$/g |
| Moisture (as packed) | 2 mass - % (max 10 mass - %) |
| Ash content | 8 mass - % |
| Chloride (acid extr) | 0.04 mass - % |
| pH | alkaline |
| Apparent density (tamped) | 545 kg/$m^3$ |
| Particle size $D_{10}$ | 4 $\mu$m |
| Particle size $D_{50}$ | 32 $\mu$m |
| Particle size $D_{90}$ | 180 $\mu$m |

We measured the pH of Norit SA 4G to be around 10, using deionised water with a pH of 5-6.

We prefer to use from 0.01 to 10 wt %, for example from 0.01 to 5 wt %, of the adsorbent or the combination of the adsorbent and the filter aid based on the weight of the plasticiser ester to be purified. More preferably we use from 0.02 to 2 wt % most preferably 0.03 to 1 wt % and in particular 0.04 to 0.3 wt %. In embodiments where the filter aid and the adsorbent, eg activated carbon are different materials, these may be separately added to the ester in steps (v) and (vi) of the process. However, we prefer to add both materials at step (v), preferably as mixture. In this case the mixture may contain, for example, from 90 to 30 parts by weight of the filter aid and from 10 to 70 parts by weight of the adsorbent. Preferably the mixture contains from 70 to 30 parts by weight of the filter aid and from 30 to 70 parts by weight of the adsorbent. More preferably the mixture contains from 60 to 40 parts by weight of the filter aid and from 40 to 60 parts by weight of the absorbent. Our most preferred mixture contains from 45 to 55 parts by weight of the adsorbent and correspondingly from 55 to 45 parts by weight of filter aid. For cost reasons, a lower content of adsorbent is preferred, but as the level of adsorbent is reduced, also its effect is reduced. We therefore generally prefer to use at least 30 parts by weight of adsorbent in the mixture. However, when the filter aid is particularly effective by itself, the adsorbent eg active carbon may also be reduced to as low as, for example, 10, 15 or 20 parts by weight of the mixture, with correspondingly 90, 85 or 80 parts by weight of the filter aid.

We have found that use of the mixture has the additional benefit that it improves the stability of the plasticiser to ultra violet light, which is particularly important for plasticisers that are used in polyvinyl chloride formulations that are to provide electrical insulation in areas that are heavily exposed to daylight and particularly to direct sunlight. Low concentrations of light ends and odour formers are also important when the products are to be used in a confined space such as a space capsule, an aeroplane or truck cabin, a car interior or a green house. When a mixture is used the filter aid and the adsorbent (when different) may be added separately to the plasticiser ester although we prefer that they be added as a mixture as this enables the use of a single injection position in the purification vessel.

The adsorbent and/or the filter aid are preferably added continuously to the plasticiser flowing through a stirred vessel which is optionally provided with baffles to enhance mixing.

The preferred treatment with adsorbent depends upon the nature of the plasticiser ester and the nature of the adsorbent and/or the filter aid. However, we prefer that the treatment be performed at a temperature in the range 20 to 180°C, more preferably 50 to 150°C, most preferably 80 to 120°C e.g. 80 to 115°C and in particular 90 or 100 to 110°C. In particular we have found that a temperature in the range 80 to 120°C e.g. 80 to 115°C is especially useful in the treatment of $C_8$ to $C_{13}$ dialkyl phthalates. It is preferred that the treatment be a continuous process with the plasticiser having a residence time of at least one hour, for example from one to eight hours, preferably from one to two hours in the treatment drum.

The invention is illustrated by reference to the following Examples, all the tests in which were performed on stripped plasticiser that was produced according to steps (i) to (iv) of the process of the invention.

The LVR values were measured with a Pilot cell for liquid insulating materials Type 2905 from Tettex Instruments and using a Hewlett Packard High Resistance meter HP 4329A. The cell and the samples to be measured were stored overnight in the room where the measurement took place, and which had a controlled atmosphere, so that samples and cell had the same temperature. To carry out the measurement the cell was rinsed at least twice with the product to be measured, and then filled again. The cell was then charged with a DC tension of 500V for 120 seconds after which the resistivity was measured. A thermometer was inserted into the sample bottle to determine the plasticiser temperature with an accuracy of +/- 0.1 °C. The cell was then discharged, emptied and refilled for a second measurement. If the two measurements were not close, i.e. within 10% of each other, it was assumed that the cell was not yet sufficiently clean. In such a case, the procedure is repeated until successive measurements come out close. The measured value then becomes the average of the last few measurements that were sufficiently close. Because the LVR is highly temperature dependent (the lower the temperature, the higher the LVR), the results were reported at a standard temperature, the reported value being obtained by recalculating the measured value to a standard temperature of 20.0°C by applying the following correction formula:

$$\text{Reported value at } 20°C = \text{measured value at } T°C * \{ (1.05) \exp (T - 20.0) \}$$

The carbonyl numbers were determined by reaction of the carbonyl functions in the sample with hydroxylammoniumchloride to form an oxime, followed by the potentiometric titration of the liberated hydrochloric acid with an alcoholic solution of tetra-n-butyl ammonium hydroxide, a method that is based on ISO 1843. The detailed procedure was as follows:

A hydroxylammoniumchloride solution was prepared by dissolving 20.00 g of hydroxylammoniumchoride [Merck 4616] into 100 g of distilled water and further diluted with ethanol p.a. grade [Merck 983] to a volume of 1000.0 ml. A sample of about 30 grams of plasticiser was weighed, with an accuracy of +/-0.1 mg, into a flat bottom flask containing 10.00 ml of this hydroxylammoniumchloride solution. Also, a blank flask was prepared containing only 10.00 ml of hydroxylammoniumchloride solution. Then 25 ml of a solution made of 200 ml distilled water and 800 ml ethanol p.a. grade was added to each flat bottom flask. Reflux coolers were connected to each flask and the solution was boiled for a total time of 45 minutes, including heating up time. With the reflux cooler connected and the flask removed from the heating mantle, the flasks were allowed to cool for 15 minutes. Each reflux cooler was then rinsed with 10 ml of ethanol p.a. into the flask, and the content of the flasks was then quantitatively transferred into 250 ml plastic beakers, using ethanol p.a. for rinsing and for topping up to a total volume of 100 ml. The content of the plastic beakers was then submitted to potentiometric titration with a 0.1000 N solution of tetra-n-butylammonium hydroxide (TBAH) in 2-propanol/methanol [Merck 9162]. For this purpose we used a Metrohm titroprocessor 670 equipped with a Metrohm sample changer 674 and a Metrohm Dosimat 665 with a 20.00 ml burette. As electrodes we used a Metrohm Platinum electrode 6.0302.110, a Metrohm Ag/AgCl reference electrode 6.0729.110 and a Metrohm separate pH glass electrode 6.0130.100.

The titrated volumes (in ml) of TBAH Vl and V0 were established at the inflection points of the titration of respectively the sample and of the blank, if any for the latter. The carbonyl number, expressed in mg KOH/g of the sample was then calculated as:

$$\text{Carbonyl Number} = \frac{(V1 - V0) * 56.1 * 0.1000 \, N}{\text{Sample weight in gram}}$$

The light ends and intermediates content were measured by Gas Chromatography as follows: about 3 g of plasticiser sample, measured with 0.1 mg accuracy, was placed into a 10 ml vial and, using a 10 $\mu$l GC syringe, 5.0 $\mu$l of n-C16 hydrocarbon was added as an internal standard (density: 0.773). The vial was then closed with a snapcap. The mixture in the vial was then shaken vigorously to homogenize it, while contact was avoided between the liquid in the vial and the septum of the snapcap. The concentration of internal standard is then the weight of the added standard (3.865 mg) divided by the weight of the plasticizer sample.

The analytical instrument used was a HP6890 Series gaschromatograph with direct injection. The column was a HP-1 (crosslinked methyl silicone gum, internal diameter 0.53 mm, length 30 m and a film thickness of 0.88 $\mu$m). The conditions for the analytical test were as follows:

| | |
|---|---|
| Oven temp: | programmed to ramp up from 100°C to 320°C at a rate of 8 degrees C/min, where it then is kept for another 25 minutes. |
| Injector: | Temp:300°C Pressure : 77.2 kPa gauge (11.2 psig) helium atmosphere |
| Carrier gas: | Helium, flow controlled at 14 ml/min (at 100°C) |
| FID Detector: | Temp: 320°C $H_2$ flow: 35 ml/min Air flow: 350 ml/min Make up gas to the detector (helium): 11 ml/min (at 100°C) |
| Sample size: | 0.1-0.2 $\mu$l |

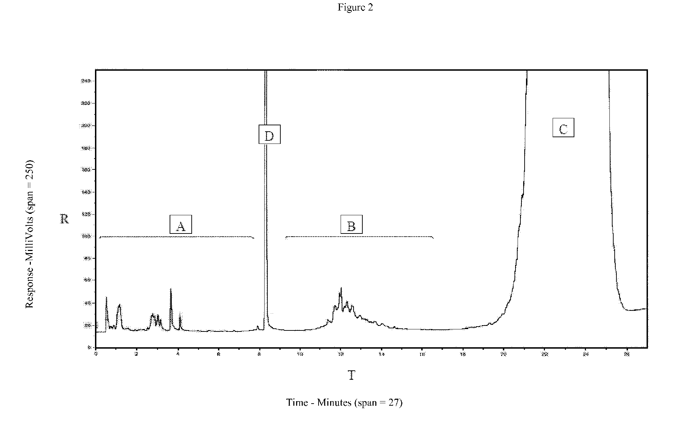

Figure 2 shows a typical DIDP GC spectrum having a horizontal axis T indicating time in minutes (span 27 minutes); and a vertical axis R indicating response in millivolts (span 250 mv). The spectrum shows, besides the plasticiser fingerprint itself (indicated as C) and the normal hexadecane (C16) internal standard peak (indicated as D), two more groups of peaks:

- the light ends are shown by a group of peaks eluting before the C16 standard (and thus with a retention time of less than 8 min). They comprise olefins, paraffins, aldehydes, alcohols, formates, phthalide and phthalic acid, and are marked as A on Figure 2
- the intermediates are shown by a group of peaks eluting between the n-C16 internal standard peak and the peak of the plasticiser ester itself (retention time above 8 min but below that of the plasticiser). They comprise dialkyl ethers, hemicacetals, benzoates and other esters, and other materials, and are marked as B on Figure 2

The concentration of the light ends is obtained from the spectrum according to the following equation:

$$\text{light ends ppm} = \frac{\text{area light ends x wtppm } C_{16}}{\text{area } C_{16}}$$

in which "wtppm $C_{16}$" is the concentration of the normal hexadecane standard in the sample, expressed in ppm weight, "area light ends" is the sum of the areas of all the peaks making up the light ends components in region indicated as A of Figure 2, and "area $C_{16}$" is the area of the normal hexadecane internal standard peak in the GC spectrum.

The concentration of the intermediates is obtained in a similar way by the integration of the area under the peaks in the region indicated as B in Figure 2.

EXAMPLE 1

The products as indicated in Table 1 were each separately placed in a glass beaker with a magnetic stirrer and heated to 100°C while stirring. When that temperature was reached, the treating agent or mixture as indicated in Table 1 was added into the beaker. The amount of treating agent or mixture used was always about 5% by weight relative to the amount of liquid in the beaker. After one minute, the treatment time as indicated in Table 1 was started. After the treatment, the stirring was stopped and the content of the beaker was filtered immediately. The samples were filtered twice, each time using 2 layers of filter paper (Machery & Nagel MN 616) placed in a Büchner funnel. The filtration was assisted by pulling vacuum on the filter flask on which the filter funnel was placed.

The filtered samples were then stored in glass bottles and allowed to cool to room temperature. The LVR-value was measured the following day. GC and Odour tests were also carried out. The results are summarized in Table 1.

The experiments were performed in three groups. The same starting material was used for all the experiments in each group, and the properties of that starting material is shown in the first line of each group in Table 1 ("Treating Agent or Mixture" indicated as "None"). In the final column of Table 1, the combined wtppm of the light ends and intermediates is designated as "Total Lights".

In Table 1:

- The Norit used was Norit SA4G.

- The compositions of both the frieBE and the Norit SA 4 G were as described hereinbefore.

- Tonsil 314 is Tonsil standard 314 FF highly active bleaching earth, a clay marketed by Süd-Chemie AG and made by acid activation of natural calcium bentonite.

- Ton 02/B is Galleon Earth, an acid activated clay available from Ashapura Volclay Limited and made by treating high purity montmorillonite clay (bentonite) with sulphuric acid.

- DINP = di-isononyl phthalate.

- DOP = di-octyl phthalate (where di-octyl is di-2-ethylhexyl).

- Odour designations are:

- = no odour

+ = slight odour

++ = strong odour

+++ = very strong odour

Table 1

| Plasticiser | Treating Agent or Mixture | pH [as provided by vendor] | Time hours | LVR @20°C Ohm.cm (x $10^{12}$) | Odour | Carbonyl Number mg KOH/g | Light Ends wtppm | Intermediates wtppm | Total Lights wtppm |
|---|---|---|---|---|---|---|---|---|---|
| DINP | None | | | 0.32 | - | 0.10 | 488 | 503 | 991 |
| DINP | frieBE | 8.3 | 8 | 1.54 | ++ | 0.15 | 363 | 416 | 779 |
| DINP | Ton 02/B | 3 | 8 | 0.76 | ++ | 0.09 | 905 | 387 | 1292 |
| DINP | Norit | | 8 | 1.99 | - | 0 | 455 | 418 | 873 |
| DINP | Tonsil 314 | 2.2-6.1 | 9 | 0.63 | +++ | 0.21 | 966 | 503 | 1469 |
| DINP | frieBE/Norit 90/10 | | 8 | 2.03 | + | 0.12 | 406 | 316 | 722 |
| | | | | | | | | | |
| DINP | None | | | 0.52 | - | 0.1 | 598 | 434 | 1032 |
| DINP | frieBE/Norit 50/50 | | 8 | 2.78 | - | 0 | 448 | 336 | 784 |
| | | | | | | | | | |
| DOP | None | | | 0.25 | - | 0 | 514 | 599 | 1113 |
| DOP | Tonsil 314 | 2.2-6.1 | 8 | 0.09 | ++ | 0.24 | 768 | 569 | 1427 |
| DOP | frieBE/Norit 50/50 | | 8 | 1.17 | - | 0 | 387 | 427 | 814 |

The results reported in Table 1 show that a treatment with clay alone (frieBE, Ton 02/B, Tonsil 314) while improving the LVR, can also cause the development of odour, which is associated with an increase in light ends concentration and/or an increase in the carbonyl number. A treatment with active carbon alone (Norit) improves LVR without showing those drawbacks. It can even show a reduction in light ends and/or carbonyl number, however it may be less cost effective and somewhat difficult to filter. A mixture of active carbon with clay as a filter aid (frieBE/Norit) is easier to filter and gives much improved LVR. The 90/10 ratio clay/active carbon mixture gave a product with a slight odour; the 50/50 ratio mixtures gave an odour-free product.

EXAMPLE 2

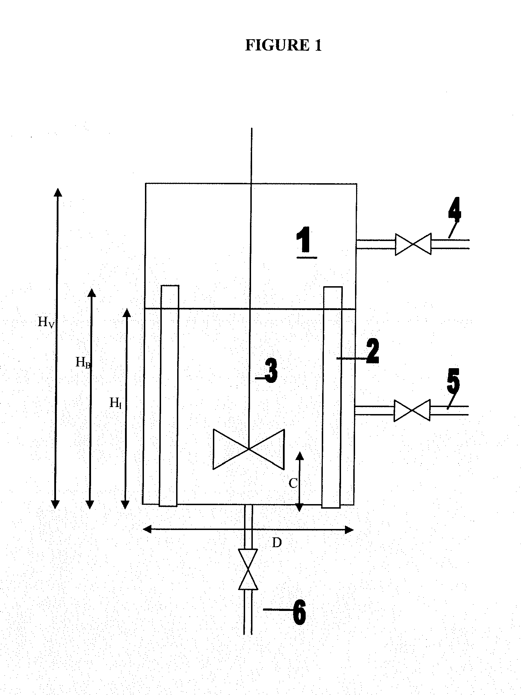

Various samples of di-isodecyl phthalate (DIDP) were treated with 0.2 %wt of a specific treating agent or mixture for 1 hour in the vessel shown in Figure 1. The vessel is a vertical cylinder 1 with a diameter D of 300 mm and a height $H_v$ of 400 mm. Inside the vessel, four baffles 2 each 25 mm wide are positioned every 90 degrees around the vertical wall of the vessel, perpendicular to the wall and leaving a 5 mm gap from the inner wall of the vessel. The baffles have a height $H_B$ of 300 mm. The height of the liquid $H_L$ used was always 295 mm, so the liquid volume in the vessel was typically 20.85 litre.

An impeller 3 of type A310 (as supplied by LIGHTNIN) is provided for stirring the content of the vessel. It is positioned along the vessel axis at a height above the vessel bottom of C = D/4 = 75 mm. The motor for driving the impeller was a Heidolph Type 743.00, with 30 W power. The impeller speed as measured was around 335 rpm.

The vessel is equipped with three electrical heating jackets, and the temperature is controlled via a control box. The vessel has a connection 4 for inlet of blanketing nitrogen, a sampling line 5, and a drain connection 6 for draining the vessel.

The vessel was filled with the stripped DIDP and a constant flow of nitrogen was established for blanketing. Stirring was established at a speed of 335 rpm. The vessel content was then heated to 105°C, after which the treating agent or mixture as indicated in Table 2 was added to the vessel. One minute later, the treatment time was started. Samples were taken after one hour and filtered immediately, as described in Example 1. Again the filtered samples were stored in glass bottles and allowed to cool to room temperature. The LVR was measured the following day. Also GC runs were carried out on the samples. The results are summarized in Tables 2a and 2b.

In Tables 2a and 2b, the materials that are also reported in Table 1 are as described in the discussion of Table 1. Of those materials reported for the first time in Tables 2a and 2b:

- Tonsil 412 FF and Tonsil 4121 FF are highly active bleaching earths from Süd-Chemie AG, made by acid activation of calcium bentonite followed by blending with, respectively, 5 wt.% and 10 wt.% activated carbon.
- Norit is Norit SA4G, as previously described.
- Bent Actigel is a bleaching agent that is a mixture of acid activated bentonite with activated carbon, available from BENSAN Activated Bentonite Company.
- Suprefast FF EX is a bleaching agent that is an acid activated bentonite, available from BENSAN Activated Bentonite Company.
- Norit SA 4 PAH is a steam activated carbon powder developed for bleaching of edible oils and fats. It is especially suitable for removal of polycyclic aromatic hydrocarbons (PAH) from oils such as coconut and sunflower oil.
- Tonsil 3191 FF, marketed by Süd-Chemie AG, is a naturally active bleaching earth, made by treatment of calcium bentonite by a proprietary method. The main constituent of bentonite is the mineral montmorillonite, an aluminium hydrosilicate in which the proportion of silicic acid to alumina is about 4:1.
- Volcansil RN-70 and Volcansil D.E./11 are hydrated alumino-magnesium silicates, also called alumino-magnesium silicates or Fuller's Earth, available from BENESA, Bentonitas Especiales. S.A. The materials are defined as calcium bentonites and are clays that are not acid activated. The two materials have similar physico-chemical properties, and differ primarily in terms of particle size distribution. Their main commercial uses are in the treatment of olive oils, wines and beers.
- The Light Ends/Intermediates columns marked ** in Tables 2a and 2b list "light ends" content first, then "intermediates" content (each measured from the GC spectrum analysis), and then the sum of these two is shown as a sub-total.
- The carbonyl numbers of the initial DIDPs and of the DIDPs treated with neutral or alkaline agents showed little variation; all were below 0.2 mg KOH/g and most were close to zero. By way of example, the carbonyl numbers of the DIDP treated using the final four treating agents/mixtures shown in Table 2b were as set out in Table 3.

Table 2a

| Treating Agent or Mixture | pH [as measured on the solid] | LVR Ohm.cm (x $10^{12}$) | | | Light Ends Intermediates wt ppm ** | |
|---|---|---|---|---|---|---|
| | | Initial | Treated | % increase | Initial | Treated |
| Tonsil 4121 FF | 4.0 | 0.78 | 1.01 | 29 | 456 | 490 |
| | | | | | 600 | 458 |
| | | | | | 1056 | 948 |
| Tonsil 412 FF | 4.4 | 0.54 | 0.66 | 22 | 456 | 645 |
| | | | | | 600 | 427 |
| | | | | | 1056 | 1072 |
| NORIT active carbon | 10.7 | 0.48 | 2.19 | 355 | 456 | 289 |
| | | | | | 600 | 545 |
| | | | | | 1056 | 834 |
| Bent Actigel (20% carbon) | 4.3 | 0.48 | 1.42 | 196 | 456 | 558 |
| | | | | | 600 | 412 |
| | | | | | 1056 | 970 |
| Suprefast FF EX | 4.0 | 0.61 | 0.83 | 37 | 375 | 430 |
| | | | | | 536 | 435 |
| | | | | | 911 | 865 |

Table 2b

| Treating Agent or Mixture | pH [as measured on the solid] | LVR Ohm.cm (x $10^{12}$) | | | Light Ends Intermediates wt ppm ** | |
|---|---|---|---|---|---|---|
| | | Initial | Treated | % increase | Initial | Treated |
| 90% Tonsil 314 FF 10% Norit SA 4G | 7.4 | 0.75 | 2.25 | 201 | 363 | 560 |
| | | | | | 630 | 622 |
| | | | | | 993 | 1182 |
| 90% Tonsil 314 FF 10% Norit SA4PAH | 7.3 | 0.75 | 2.72 | 264 | 363 | 320 |
| | | | | | 630 | 576 |
| | | | | | 993 | 896 |
| 50% frieBe 50% Norit SA 4G | 10.3 | 0.82 | 3.06 | 289 | 363 | 302 |
| | | | | | 630 | 616 |
| | | | | | 993 | 918 |
| Tonsil 3191 FF | 7.8 | 0.36 | 0.79 | 116 | 681 | 850 |
| | | | | | 813 | 620 |
| | | | | | 1494 | 1470 |
| Volcansil RN-70 | 7.2 | 0.33 | 1.39 | 327 | 681 | 877 |
| | | | | | 813 | 628 |
| | | | | | 1494 | 1505 |
| Volcansil D.E./11 | 7.3 | 0.33 | 0.77 | 136 | 681 | 713 |
| | | | | | 813 | 688 |
| | | | | | 1494 | 1401 |

Table 3

| Treating Agent or Mixture | Carbonyl Number of DIDP (mg KOH/g) |
|---|---|
| Untreated | 0.00 |

(continued)

| Treating Agent or Mixture | Carbonyl Number of DIDP (mg KOH/g) |
|---|---|
| frieBE/Norit SA 4G (50/50) | 0.00 |
| | |
| Untreated | 0.09 |
| Tonsil 3191 FF | 0.09 |
| Volcansil RN-70 | 0.09 |
| Volcansil D.E./11 | 0.08 |

The results reported in Tables 2a, 2b and 3 show that treatment with clays that have an acidic pH (Tonsil 4121 FF and 412 FF, Suprefast FF EX) give only moderate LVR improvements and cause a reduction of the content of intermediates, which goes together with an increase of the light ends content. Treatment with only active carbon (Norit), with an alkaline pH, gives an excellent LVR improvement, and is capable of removing a part of the light ends, while leaving the intermediates content practically intact. Treatment with an acidic mixture of clay and active carbon (Bent Actigel at 20% carbon) gives a good LVR improvement together with a reduction in total of light ends/intermediates (light ends is increased, intermediates decreased).

Treatment with a clay (Tonsil 3191 FF, Volcansil RN-70 and D.E./11), or a mixture of clay and active carbon (Tonsil 314 FF with 10% Norit SA 4G or SA4PAH), that has a pH of about neutral, gives good-to-excellent LVR improvements. The intermediates content can be somewhat reduced and the light ends content can increase, but the carbonyl number is not increased. Using a higher active carbon content in such a solids mixture, which also makes the pH of the solid more alkaline, gives an excellent LVR improvement with substantially no change in carbonyl number and minimal changes in intermediates content and light ends content.

The plasticiser treatment vessel used in Example 2 is laboratory scale. A typical scaled up vessel for commercial plasticiser purification would be for example a cylindrical vertical drum with korbbogen heads having dimensions: D = 4000 mm and $H_v$=5400 mm. Such a vessel would contain four vertical baffles at 90° around the vertical wall, with a width of 333 mm and a height 4900 mm, leaving a gap of 55 mm with the vessel wall. Liquid level in the vessel would be 4800 mm max. (All these heights considered as expressed from bottom tangent line up.) The vessel would be fitted with an impeller such as an HE-3 having a 1626 mm diameter and a 137 mm height. The height of the impeller from the bottom tangent line would be 123 mm and the speed of the impeller would be 86 rpm.

## Claims

1. A di-alkyl phthalate **characterised by** a carbonyl number below 0.2 mg KOH/g, a light ends content of less than 1000 ppm wt, and a liquid volume resistivity (LVR) that is:

   i) greater than 0.3 x $10^{12}$ ohm.cm in the case where di-alkyl is di-2-ethyl hexyl;
   ii) greater than 0.6 x $10^{12}$ ohm.cm in the case where di-alkyl is di-isononyl; and
   iii) greater than 1.35 x $10^{12}$ ohm.cm in the case where di-alkyl is di-isodecyl.

2. The phthalate of claim 1 wherein the LVR (in units of $10^{12}$ ohm.cm) is

   i) greater than 0.5 or greater than 0.8 or greater than 1.1 in the case where di-alkyl is di-2-ethyl hexyl;
   ii) greater than 0.7 or greater than 1.0 or greater than 2.0 in the case where di-alkyl is di-isononyl; and
   iii) greater than 2.0 or greater than 2.5 or greater than 3.0 in the case where di-alkyl is di-isodecyl.

3. The phthalate of claim 1 or 2 wherein the carbonyl number is 0.1 mgKOH/g or less.

4. The phthalate of claim 1, 2 or 3 wherein the light ends content is below 600 ppm wt.

5. The phthalate of claim 1, 2, 3 or 4 containing intermediates, wherein the intermediates content is below 750 ppm wt.

6. The phthalate of claim 5 having a combined light ends and intermediates content below 1000 ppm wt.

**7.** A polyvinyl chloride composition comprising polyvinyl chloride plasticised with a phthalate according to any of claims 1 to 6.

**8.** The use of a polyvinyl chloride composition according to claim 7 for wire and cable insulation.

**FIGURE 1**

Figure 2

Europäisches
Patentamt

European
Patent Office

Office européen
des brevets

# EUROPEAN SEARCH REPORT

Application Number

EP 10 18 9204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 5 880 310 A (AGEISHI ET AL) 9 March 1999 (1999-03-09) * column 2, lines 40-50; column 2, line 60 - column 3, line 4; column 3, lines 39-45; column 4, line 37 - column 5, line 54; Examples; Tables 1, 2 and 3; claims * | 1-8 | INV. C07C69/80 C07C67/08 C07C67/48 H01B3/44 |
| X | GB 1 096 917 A (IMPERIAL CHEMICAL INDUSTRIES LIMITED) 29 December 1967 (1967-12-29) * page 1, lines 64-66; Examples I to IV; claims * | 1-8 | |
| X | US 5 798 319 A (SCHLOSBERG ET AL) 25 August 1998 (1998-08-25) * column 2, lines 43-53; column 4, lines 34-45; column 5, line 23 - column 6, line 20; column 15, Table 1, lines 36-39; claims, in particular, claims 1-9; abstract * | 1-8 | |
| X | US 4 284 793 A (SAGARA ET AL) 18 August 1981 (1981-08-18) * Example 1, 4; claims * | 1-8 | TECHNICAL FIELDS SEARCHED (IPC) C07C H01B |
| X | US 6 437 170 B1 (THIL LUCIEN ET AL) 20 August 2002 (2002-08-20) * column 2, lines 25-31; column 7, lines 25-45; column 8, lines 3-6; column 12, line3-32; column 12, line 51 - column 13, line 3; claims * | 1-8 | |
| X | PATENT ABSTRACTS OF JAPAN vol. 2000, no. 24, 11 May 2001 (2001-05-11) & JP 2001, 206866, A, (MITSUBISHI CHEMICALS CORP), 31 July 2001 (2001-07-31) * abstract * | 1-8 | |

The present search report has been drawn up for all claims

| Place of search | Date of completion of the search | Examiner |
|---|---|---|
| Munich | 15 April 2011 | Sen, Alina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 18 9204

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2011

| Patent document cited in search report | | Publication date | Patent family member(s) | | Publication date |
|---|---|---|---|---|---|
| US 5880310 | A | 09-03-1999 | NONE | | |
| GB 1096917 | A | 29-12-1967 | BE | 684831 A | 30-01-1967 |
| | | | DE | 1270029 B | 12-06-1968 |
| | | | SE | 309774 B | 08-04-1969 |
| US 5798319 | A | 25-08-1998 | AU | 720560 B2 | 01-06-2000 |
| | | | AU | 2242797 A | 11-08-1997 |
| | | | BR | 9706996 A | 20-07-1999 |
| | | | CA | 2242389 A1 | 24-07-1997 |
| | | | CN | 1211236 A | 17-03-1999 |
| | | | EP | 0880495 A1 | 02-12-1998 |
| | | | JP | 2000516970 T | 19-12-2000 |
| | | | NO | 983257 A | 15-07-1998 |
| | | | WO | 9726238 A1 | 24-07-1997 |
| US 4284793 | A | 18-08-1981 | JP | 1368046 C | 11-03-1987 |
| | | | JP | 54076517 A | 19-06-1979 |
| | | | JP | 60054945 B | 03-12-1985 |
| US 6437170 | B1 | 20-08-2002 | AT | 261929 T | 15-04-2004 |
| | | | AU | 778207 B2 | 25-11-2004 |
| | | | AU | 1387200 A | 02-11-2000 |
| | | | BR | 9917230 A | 08-01-2002 |
| | | | CA | 2367358 A1 | 26-10-2000 |
| | | | CN | 1350515 A | 22-05-2002 |
| | | | DK | 1171413 T3 | 19-07-2004 |
| | | | WO | 0063151 A1 | 26-10-2000 |
| | | | EP | 1171413 A1 | 16-01-2002 |
| | | | ES | 2217891 T3 | 01-11-2004 |
| | | | JP | 2002542217 T | 10-12-2002 |
| | | | TW | I274052 B | 21-02-2007 |
| JP 2001206866 | A | 31-07-2001 | JP | 4120123 B2 | 16-07-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5880310 A
- US 6310235 B

- WO 9417028 A